# EUROPEAN PATENT SPECIFICATION

(11) **EP 1 332 735 B1**
(45) Date of publication and mention of the grant of the patent: **08.08.2007**
(21) Application number: 03250519.0
(22) Date of filing: 28.01.2003
(51) Int. Cl.: A61F 2/34, A61F 2/38, A61F 2/40, A61L 27/16, B29C 35/08, B29C 43/16, B32B 27/16, C08J 7/18

(54) **Method of manufacture of a composite prosthetic bearing having a crosslinked articulating surface**
Herstellungsverfahren eines Komposit-Prothesenlagerelements mit einer vernetzten Gelenkfläche
Procédé de fabrication d'un palier de frottement composite prothétique avec surface d'articulation réticulée

(30) Priority: 28.01.2002 US 58495
(43) Date of publication of application: 06.08.2003
(73) Proprietor: DePuy Products, Inc., Warsaw, Indiana 46581 (US)
(72) Inventor: King, Richard, Warsaw, IN 46580 (US); Smith, Todd S., Warsaw, IN 46582 (US); McNulty, Donald E., Ft. Wayne, IN 46804 (US)
(74) Representative: Belcher, Simon James

(56) References cited:
- US-A- 5 106 545
- US-A- 6 165 220
- US-A1- 2001 026 033
- US-B1- 6 203 844

## Description

The present invention relates generally to a method for making prosthetic bearing, and more particularly to a method for making composite prosthetic bearing having a crosslinked articulating surface.

Implantable prosthetic bearings such as acetabular bearings, glenoid bearings, tibial bearings and the like have typically been constructed from polyethylene. Indeed, ultra-high molecular weight polyethylene (UHMWPE) is generally utilized in the construction of a prosthetic bearing due to its favourable characteristics in relation to the articulating surface of the bearing. Moreover, it has been determined that certain characteristics of UHMWPE may be enhanced by exposing UHMWPE to radiation such as gamma radiation. In particular, exposing UHMWPE to predetermined doses of radiation crosslinks the UHMWPE thereby increasing its wear resistance. As such, heretofore designed prosthetic bearings have been constructed of crosslinked UHMWPE in order to gain the benefits mentioned above. Techniques for crosslinking, quenching, or otherwise preparing UHMWPE are disclosed in US-5728748, US-5879400,US-6242507, US-6316158, US-6228900, US-6245276 and US-6281264.

Conventional (i.e. non-crosslinked) UHMWPE also possesses a number of favourable characteristics relating to the construction of a prosthetic bearing. For example, conventional UHMWPE possesses superior ductility, toughness, and creep resistance characteristics relative to other polymers.

A prosthetic bearing is typically designed to include structures or features which perform two primary functions. Firstly, a typical prosthetic bearing design includes an articulating or bearing surface on which either a natural bone structure or a prosthetic component articulates. Secondly, a typical prosthetic bearing design also includes locking features in the form of mechanisms such as pins, tabs, tapered posts, or the like for locking or otherwise securing the bearing to either another component associated with a prosthetic assembly (e.g., a metal shell or tray) or to the bone itself.

As described above, certain polymers may have enhanced characteristics relating to one of these primary functions of the bearing (i.e., the function of providing an articulating surface), whereas other polymers may have enhanced characteristics relating to the other primary function of the bearing (i.e., the function of locking the bearing to another component or to the bone itself). What is needed, however, is a prosthetic bearing which is constructed from polymers which have enhanced characteristics relating to both primary functions of the bearing.

Another challenge associated with implantable prosthetic bearings relates to the construction of bearings which are designed to be secured directly to the bone without the use of a metal shell or tray. For example, prosthetic bearings designed completely of polyethylene may be difficult to affix to the bone with the use of bone cement since most commonly utilized bone cements do not adhere well to polyethylene. As such, a number of bearings have heretofore been designed which attempt to overcome this problem by use of a composite material. For example, a bearing disclosed in US-5645594 includes a first layer of UHMWPE and a second layer of blended UHMWPE and polymethyl methacrylate (PMMA). PMMA is a common component in many types of bone cement. It is disclosed that the PMMA portion of the blend may be either PMMA homopolymers or PMMA copolymers. However, a blend is, by definition, non-homogeneous and is therefore often susceptible to undesirable process and product variations. What is needed therefore is a prosthetic bearing which facilitates enhanced adhesion of the bearing to bone cement when the bearing is being secured directly to bone without the use of an implanted metal shell or tray.

US-6203844 discloses a polymeric prosthesis component in which the polymer is crosslinked. The polymer is crosslinked using a chemical crosslinking agent in a reaction which is initiated by application of heat. The crosslinking agent forms bonds between the layer of the crosslinked polymer and a layer of another polymer.

US-6165220 discloses a polymeric implant component, in which the polymer is crosslinked in a surface region by exposure to electrons or by treatment with a chemical crosslinking agent.

US-6203844 discloses a polymeric prosthesis component which comprises a plurality of layers of polymeric material. An innermost layer can be formed from a crosslinked polymer, which is melt fused to an adjacent underlying layer.

The present invention provides a method of making a bearing for an orthopaedic prosthesis, which comprises:
a. exposing a first polymer to a dose of radiation so as to cause the first polymer to become crosslinked,
b. securing a layer of the first polymer, after it has been crosslinked, to a layer of a second polymer to form a composite structure which comprises the layers of the first and second polymers, in which the first layer is secured to the second layer by a process which comprises compression moulding at elevated temperature and pressure sufficient to cause the polymer layers to become bonded to one another, and
c. forming the composite into a predetermined shape.

Accordingly, the present invention provides a method for making an implantable polymer prosthetic bearing constructed of a composite material having a first layer and a second layer. The first layer has an articulating surface defined therein, whereas the second layer has an engaging surface defined therein for engaging either another prosthetic component or the bone itself. In certain embodiments, the first layer of the implantable prosthetic bearing is constructed from a crosslinked polymer such as UHMWPE, whereas the second layer of the implantable prosthetic bearing is constructed from a polymer such as UHMWPE that is either non-crosslinked or crosslinked to a lesser degree than the first layer. In such a manner, the first layer possesses mechanical properties which are advantageous in regard to the articulating surface (e.g., enhanced wear and oxidation resistance), whereas the second layer possesses mechanical properties which are advantageous in regard to the engaging surface (e.g., high ductility, toughness, and creep resistance).

The present invention relates to methods of making implantable prosthetic bearings. What is meant herein by the term "bearing" is an orthopaedic implant prosthetic bearing of any type, condition, shape, or configuration. Such bearings may be utilized in a number of joint replacement or repair procedures such as surgical procedures associated with the hip, shoulders, knees, ankles, knuckles, or any other joint. Embodiments of the invention will now be described by way of example with reference to the accompanying drawings, in which:
FIG. 1 is a perspective view of an implantable glenoid bearing prosthesis which incorporates the features of the present invention therein;
FIG. 2 is a cross sectional view taken along the line 2-2 of FIG. 1;
FIG. 3 is a perspective view of an implantable acetabular bearing prosthesis which incorporates the features of the present invention therein;
FIG. 4 is a cross sectional view taken along the line 4-4 of FIG. 3;
FIG. 5 is a perspective view of an implantable tibial bearing prosthesis which incorporates the features of the present invention therein; and
FIG. 6 is a cross sectional view taken along the line 6-6 of FIG. 5.

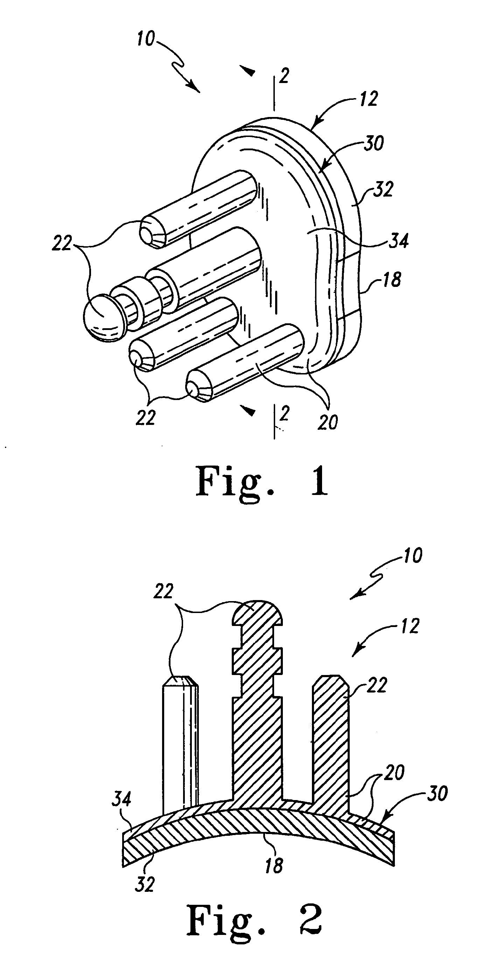

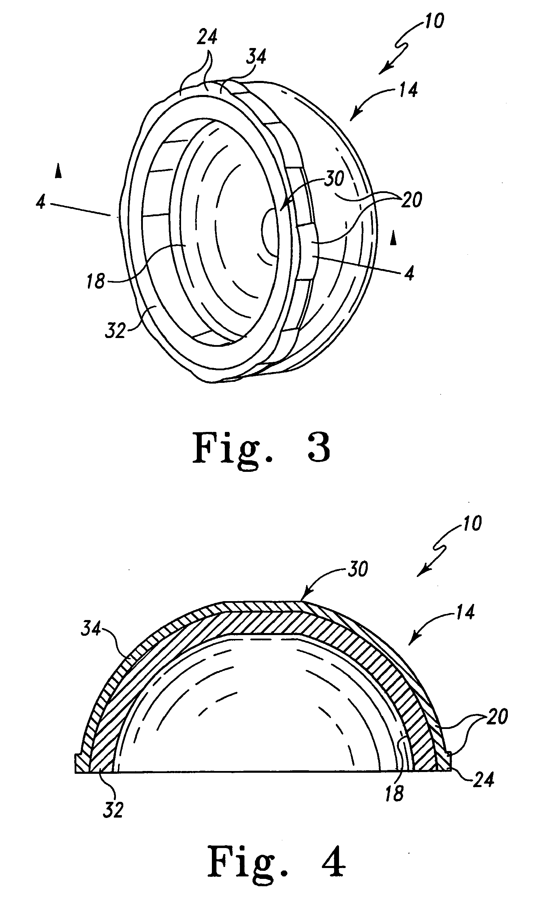

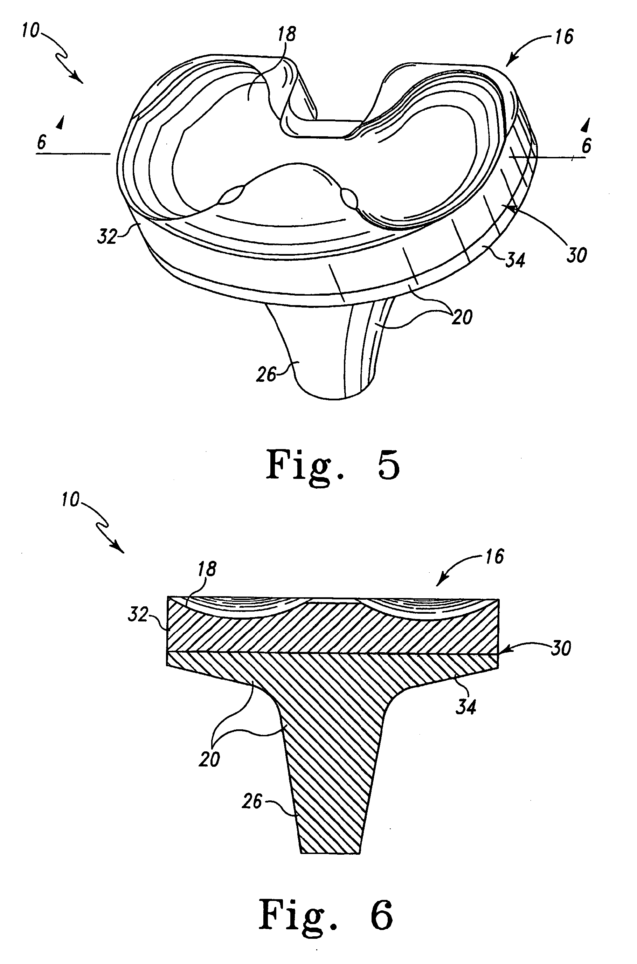

Referring now to FIGS. 1-6, there is shown a number of implantable prosthetic bearings 10 such as a glenoid bearing 12 for implantation into a glenoid of a patient (not shown), an acetabular bearing 14 for implantation into an acetabulum of a patient (not shown), and a tibial bearing 16 for implantation into a tibia of a patient (not shown). Each of the prosthetic bearings 10 includes an articulating or bearing surface 18 on which a natural or prosthetic component bears. For example, in the case of the glenoid bearing 12, a natural or prosthetic humeral head (not shown) bears on the articulating surface 18. Similarly, in the case of an acetabular bearing 14, a natural or prosthetic femoral head (not shown) bears on the articulating surface 18. Moreover, in the case of the tibial bearing 16, a pair of natural or prosthetic femoral condyles (not shown) bear on the articulating surface 18.

Each of the prosthetic bearings 10 also includes an engaging surface 20 which has a number of features defined therein for engaging either another prosthetic component or the bone into which the bearing 10 is implanted. For example, in the case of the glenoid bearing 12, a number of pins or pegs 22 may be defined in the engaging surface 20 thereof. The pegs 22 are received into a number of corresponding holes (not shown) formed in the glenoid surface of the patient. The pins 22 may be press fit or held in place with the use of bone cement. Moreover, if the glenoid bearing 12 is utilized in conjunction with an implanted metal shell, the engaging surface 20 of the bearing 12 may be configured with a tapered post (not shown) or the like for securing the glenoid bearing 12 to the shell.

In the case of the acetabular bearing 14, a number of keying tabs 24 are defined in the engaging surface 20 along the outer annular surface thereof. The keying tabs 24 are received into a number of corresponding keying slots (not shown) defined in an implanted metal acetabular shell (not shown) in order to prevent rotation of the acetabular bearing 14 relative to the implanted shell. In the case of fixation of the acetabular bearing 14 directly to the acetabulum of the patient (i.e., without the use of a metal shell), the engaging surface 20 of the bearing 14 may alternatively be configured with a number of posts or pegs (not shown) which are received into a number of corresponding holes formed in the patient's acetabulum. In such a case, the posts or pegs may be press fit or held in place with the use of bone cement. Moreover, it should be appreciated that the acetabular bearing 14 may be press fit or cemented to the patient's acetabulum without the use of posts or pegs on the engaging surface 20 thereof.

In the case of the tibial bearing 16, a tapered post 26 is defined in the engaging surface 20 thereof. The tapered post 26 is received into a corresponding tapered bore (not shown) defined in an implanted tibial tray (not shown) of a knee prosthesis (not shown). It should be appreciated that the engaging surface 20 of the tibial bearing 16 may also be configured with features to allow the tibial bearing 16 to be secured directly to the tibia without the use of an implanted tray (e.g., by use of bone cement). Moreover, it should be appreciated that a tibial bearing for use with a tibial tray may also be designed without the use of the post 26.

Each of the bearings 10 is constructed from a laminar composite 30 having a number of layers 32, 34. The first layer 32 of the laminar composite 30 is constructed of a material which possesses mechanical properties favourable for use in the construction of the articulating surface 18 (e.g., enhanced wear and oxidation resistance). The second layer 34, on the other hand, is constructed of a material which possesses mechanical properties favourable for use in the construction of the engaging surface 20 (e.g., enhanced ductility, toughness, and creep resistance). It should be appreciated that the material utilized for construction of the second layer 34 may also be selected based on the fixation type of the bearing 10. In particular, the material utilized for construction of the second layer 34 may also be varied based on, for example, whether the bearing 10 is to be utilized in conjunction with an implanted shell or directly secured to the bone (e.g., by use of bone cement).

It should be appreciated that, as used herein, the term "layer" is not intended to be limited to a "thickness" of material positioned proximate to another similarly dimensioned "thickness" of material, but rather is intended to include numerous structures, configurations, and constructions of material. For example, the term "layer" may include a portion, region, or other structure of material which is positioned proximate to another portion, region, or structure of differing material. For instance, a peg or similar structure may define a first "layer" of material, whereas a bearing body having a hole defined therein into which the peg is moulded may define a second "layer" of material. Similarly, a flange secured around the periphery of a bearing body may define a first "layer" material, whereas the bearing body itself defines the second "layer" of material.

A polymer is preferably utilized in the construction of the layers 32, 34. As used herein, the term "polymer" is intended to mean any medical grade polymeric material which may be implanted into a patient. A specific example of such a polymer is medical grade polyethylene. The term "polyethylene", as defined herein, includes polyethylene, such as a polyethylene homopolymer, high density polyethylene, high molecular weight polyethylene, high density high molecular weight polyethylene, ultrahigh molecular weight polyethylene, or any other type of polyethylene utilized in the construction of a prosthetic implant. A more specific example of such a polymer is medical grade UHMWPE. The term "polymer" is also intended to include both homopolymers and copolymers; thus, "polymer" includes a copolymer comprising ethylene and acrylate, such as methyl methacrylate, methyl acrylate, ethyl methacrylate, ethyl acrylate and butyl methacrylate. The term "polymer" also includes oriented materials, such as the materials disclosed in EP-A-1197310

The term "polymer" is also intended to include high temperature engineering polymers. Such polymers include members of the polyaryletherketone family and the polyimide family. Specific members of the polyaryletherketone family include polyetheretherketone, polyetherketone, and polyetherketoneetherketoneketone.

In one exemplary embodiment, a composite 30 is utilized in which the first polymer layer 32 of the composite 30 is constructed with a crosslinked polymer, whereas the second polymer layer 34 of the composite 30 is constructed with a non-crosslinked polymer. In a more specific exemplary embodiment, the polymer utilized in the construction of both the first polymer layer 32 and the second polymer layer 34 of the composite 30 is polyethylene. One particularly useful polyethylene for use in the construction of the first polymer layer 32 and the second polymer layer 34 of the composite 30 is UHMWPE.

As described above, a polymer may be crosslinked by, for example, exposure to radiation such as gamma radiation. As such, the first polymer layer 32 (i.e., the crosslinked polymer layer) of the composite 30 of this exemplary embodiment may be fabricated by exposing the first polymer layer 32 to gamma radiation. Such exposure may be in the exemplary range of 10 to 150 kGy. The second polymer layer 34 (i.e., the non-crosslinked polymer layer) of the composite 30 of this exemplary embodiment is not exposed to such gamma radiation. As a result, a composite 30 constructed in accordance with this exemplary embodiment provides for an articulating surface 18 constructed of a crosslinked polymer, along with an engaging surface 20 constructed of non-crosslinked polymer. In a more specific exemplary embodiment, the first polymer layer 32 (and hence the articulating surface 18 formed therein) is constructed of a crosslinked polyethylene such as crosslinked UHMWPE, whereas the second polymer layer 34 (and hence the engaging surface 20 formed therein) is constructed of a non-crosslinked polyethylene such as a non-crosslinked UHMWPE.

Such a composite structure provides a number of advantages to the design of the prosthetic bearing 10. For example, as described above, use of a crosslinked polymer such as a crosslinked polyethylene (e.g., crosslinked UHMWPE) enhances certain favourable mechanical characteristics relating to the articulating surface 18 (e.g., high wear resistance and high oxidation resistance). Moreover, use of non-crosslinked polymers such as a non-crosslinked polyethylene (e.g., non-crosslinked UHMWPE) enhances the mechanical characteristics relating to the locking or engagement of the bearing 10 to another component or to the bone itself (e.g. enhanced ductility, toughness, creep resistance). As such, a prosthetic bearing 10 constructed in accordance with this exemplary embodiment has an articulating surface 18 which possesses the favourable mechanical characteristics associated with crosslinked polymers, along with an engaging surface 20 (and associated features such as pegs, pins, posts, etcetera) which possesses the favourable mechanical characteristics associated with non-crosslinked polymers.

In another exemplary embodiment, a composite 30 is utilized in which the first polymer layer 32 of the composite 30 is constructed from a polymer which has been crosslinked to a first degree, whereas the second polymer layer 34 of the composite 30 is constructed from a polymer which has been crosslinked to a second degree. Specifically, the second polymer layer 34 is constructed of a polymer which has been crosslinked to a lesser degree than the polymer utilized in the construction of the first polymer layer 32. One way to vary the degree in which a polymer is crosslinked is to vary the dose of radiation to which it is exposed. Specifically, in a general sense, the greater the dose of radiation to which the polymer is exposed, the greater the degree in which the polymer is crosslinked. As such, in regard to the composite 30 of this exemplary embodiment, the first polymer layer 32 is exposed to a first dose of gamma radiation, whereas the second polymer layer 34 is exposed to a second, different dose of gamma radiation. In a more specific exemplary embodiment, the dose of gamma radiation to which the second polymer layer 34 is exposed is less than the dose of radiation to which the first polymer layer 32 is exposed.

Hence, in a specific implementation of the composite 30 of this exemplary embodiment, the first polymer layer 32 may be constructed from a polyethylene such as UHMWPE which has been exposed to a first dose of gamma radiation. The second polymer layer 34, on the other hand, may be constructed with a polyethylene such as UHMWPE which has been exposed to a second, different dose of gamma radiation. It should be appreciated that the dose of gamma radiation to which the polyethylene of the second polymer layer 34 is exposed is less than the dose of radiation to which the polyethylene of the first polymer layer 32 is exposed.

It should be appreciated that the second polymer layer 34 of this exemplary composite 30, although crosslinked to some degree, still possesses many of the previously described favourable mechanical characteristics relating to a non-crosslinked polymer (e.g., enhanced ductility, toughness, and creep resistance) relative to polymers crosslinked to the same degree as the first polymer layer 32. Specifically, by crosslinking the second polymer layer 34 to a lesser degree than the first polymer layer 32, the second polymer layer 34 is able to retain more of the favourable properties relating to the engaging surface 20 relative to the properties it would possess if crosslinked to the same degree as the first polymer layer 32.

As alluded to above, the material from which the second polymer layer 34 is constructed may be selected based on whether the bearing 10 is utilized in conjunction with another implanted prosthetic component or secured directly to the bone (e.g., by use of bone cement). For example, the above described use of a polyethylene (e.g., UHMWPE) in the construction of the second polymer layer 34 (albeit non-crosslinked or crosslinked to a lesser degree than the first polymer layer 32) is particularly useful in regard to the design of bearings 10 which are utilized in conjunction with an implanted shell or tray component. However, in regard to the design of bearings 10 which are secured directly to the bone, other types of materials may be desirable in order to increase the adhesion of the bearing 10 to bone cement.

For example, in another exemplary embodiment, the second polymer layer 34 may be constructed of a copolymer which provides for adhesion to both the polymer associated with the first polymer layer 32 and bone cement. For instance, in the case of when a polymer such as polyethylene is utilized in the construction of the first polymer layer 32, a copolymer comprising ethylene and an acrylate may be utilized. It should be appreciated that, as used herein, the term "acrylate" when utilized generally (as opposed to when used in regard to the name of a specific compound or monomer) is intended to mean any compound possessing an α,β- unsaturated (i.e., 2-unsaturated) ester moiety. Examples of such α,β- unsaturated esters include methyl acrylate, ethyl acrylate, methyl methacrylate, ethyl methacrylate, butyl methacrylate, and the like.

Use of such an ethylene-acrylate copolymer provides a number of advantages. Firstly, the ethylene portion of the copolymer is particularly well suited for adhering to the polymer (e.g., polyethylene) of the first polymer layer 32 during fusion of the first polymer layer 32 and the second polymer layer 34 to one another. On the other hand, the acrylate portion of the copolymer is particularly well suited for adhesion to bone cement such as bone cement that includes poly methyl methacrylate (PMMA). In an exemplary embodiment, the ethylene-acrylate copolymer is 5-60% acrylate on a molar basis. In a more specific exemplary embodiment, the ethylene-acrylate copolymer is 15-35% acrylate on a molar basis.

In a specific exemplary embodiment, an ethylene-methyl methacrylate copolymer may be utilized in the construction of the second polymer layer 34. In another specific exemplary embodiment, an ethylene-methyl acrylate copolymer may be utilized in the construction of the second polymer layer 34. In a further specific exemplary embodiment, an ethylene-ethyl acrylate copolymer may be utilized in the construction of the second polymer layer 34. Moreover, in another specific exemplary embodiment, an ethylene-butyl methacrylate copolymer may be utilized in the construction of the second polymer layer 34.

As indicated above, the composite prosthetic bearing 10 may also be designed to be press fit to another implanted prosthetic bearing component. For such a press-fit application, the second layer of polymer 34 may include a material with properties desirable for such an application. For example, the second layer of polymer 34 may include an UHMWPE, an ethylene homopolymer or an ethylene copolymer. However, it should be understood that the invention is not limited to a particular polymer material for the second layer of polymer 34, and may encompass newly developed materials with properties desirable for the application, unless the claims expressly call for a particular material.

Moreover, it should also be appreciated that the ethylene-acrylate copolymers mentioned above that might be used in the construction of the second polymer layer 34 may be utilized in conjunction with any number of different types of polyethylene-based first polymer layers 32. For example, the first polymer layer 32 may be constructed of UHMWPE. As a further example, the crosslinked UHMWPE of either layer 32, 34 may be an oriented material such as the materials described in EP-A-1197310. The first layer of polymer 32 may also comprise a crosslinked ethylene homopolymer. And although crosslinked polymers are believed at present to provide superior wear resistance and oxidation resistance for the articulating surface in orthopaedic implants, new materials may be developed in the future with improved properties. Accordingly, the present invention is not limited to any particular material, and may encompass newly developed materials, unless a particular material is expressly set forth in the claims. In one specific exemplary embodiment, a bearing 10 is constructed with a composite 30 having a first polymer layer 32 constructed with crosslinked UHMWPE, along with a second polymer layer 34 constructed with an ethylene-acrylate copolymer.

The laminar composites 30 of the present invention may be fabricated by any technique which produces the features mentioned above. One exemplary manner for constructing the laminar composites 30 of the present invention is by use of compression moulding techniques. For example, material from which the first polymer layer 32 is to be constructed is placed in a mould, along with material from which the second polymer layer 34 is to be constructed. Thereafter, the two materials are compression moulded to one another under process parameters which cause the second material (i.e., the material from which the second polymer layer 34 is constructed) to be molten and fused to the first material thereby creating the composite 30. It should also be appreciated that the mould may be configured so as to not only fuse the two materials to one another, but also form the composite 30 into the predetermined shape associated with a prosthetic bearing 10 (e.g., the glenoid bearing 12, the acetabular bearing 14, or the tibial bearing 16). In essence, the compression moulding process not only creates the laminar composite 30 (i.e., fuses the two materials to one another), but also forms the resulting composite 30 into the desired, predetermined shape of a bearing 10.

The starting materials (e.g., polymers such as polyethylene) for use in the moulding process may be provided in a number of different forms. For example, each of the starting materials may be provided as a preform. What is meant herein by the term "preform" is an article that has been consolidated, such as by ram extrusion or compression moulding of polymer resin particles, into rods, sheets, blocks, slabs, or the like. The term "preform" also includes a preform "puck" which may be prepared by intermediate machining of a commercially available preform. Polymer preforms such as polyethylene preforms may be provided in a number of different pre-treated or preconditioned variations.

The starting materials (e.g., polymers and copolymers) may also be provided as powders. What is meant herein by the term "powder" is resin particles. Similarly to as described above in regard to preforms, powders may be provided in a number of different pre-treated or preconditioned variations.

The starting materials (e.g., polymers and copolymers) may also be provided as porous structures. What is meant herein by the term "porous structure" is a structure of compacted resin particles. The porous structure may take many forms including blocks or pucks. However, unlike preforms, a porous structure is constructed of unfused or partially fused resin particles. The porous structure may be provided in varying degrees of porosity and may include crosslinked or non-crosslinked (e.g., irradiated or non-irradiated) resin particles. In the case of a porous structure that is crosslinked via irradiation, the resin particles are typically compacted together prior to exposure to gamma or other types of radiation. However, it should be appreciated that the resin particles may be irradiated prior to compaction, if desired.

It should be appreciated that the starting materials (e.g., the preforms, powders, or porous structures) may be "pre-irradiated", "pre-quenched", or otherwise preconditioned prior to use thereof. In particular, it may be desirable for a manufacturer of prosthetic bearings to purchase material (e.g. polyethylene) which has been irradiated (or otherwise crosslinked), pre-quenched, or otherwise preconditioned by a commercial supplier or other manufacturer of the material. Such "out-sourcing" of preconditioning processes is contemplated for use in the processes described herein.

In addition to the forms of starting materials described above, the starting material may be in the form of a sheet or film, particularly where the layer of polymer comprises a copolymer of ethylene and acrylate.

In regard to fabrication of a composite 30 in which the first polymer layer 32 is constructed of crosslinked polymer and the second polymer layer 34 is constructed of non-crosslinked polymer, a number of fabrication processes may be utilized. Firstly, a preform of crosslinked polymer (i.e., pre-irradiated) may be placed in a mould, along with a preform of polymer which is non-crosslinked (i.e., non-irradiated). Thereafter, the two preforms are compression moulded to one another under process parameters which cause the non-crosslinked preform of polymer to be molten and fused to the preform of crosslinked polymer. It should also be appreciated that during such a moulding process, the resultant composite 30 formed by the fusing of the two preforms to one another is contemporaneously formed into the predetermined shape associated with a prosthetic bearing 10 (e.g., the glenoid bearing 12, the acetabular bearing 14, or the tibial bearing 16). In an exemplary implementation of this process, a preform of a crosslinked polyethylene such as crosslinked UHMWPE is compression moulded to a preform of a non-crosslinked polyethylene such as non-crosslinked UHMWPE.

Such a composite 30 (i.e., a first polymer layer 32 constructed of crosslinked polymer and a second polymer layer 34 constructed of non-crosslinked polymer) may also be fabricated by the use of polymer powders or porous structures. For example, a preform of crosslinked polymer (i.e., pre-irradiated) may be placed in a mould, along with polymer powder which is non-crosslinked (i.e., non-irradiated). Thereafter, the two materials are compression moulded to one another under process parameters which cause the non-crosslinked polymer powder to be molten and fused to the preform of crosslinked polymer. The composite 30 formed by the fusing of the two materials (i.e., the crosslinked preform and the non-crosslinked powder) to one another may be contemporaneously formed into the predetermined shape associated with a prosthetic bearing 10 (e.g., the glenoid bearing 12, the acetabular bearing 14, or the tibial bearing 16). In an exemplary implementation of this process, the crosslinked preform may be provided as a crosslinked polyethylene preform such as a crosslinked UHMWPE preform, whereas the non-crosslinked powder may be provided as a non-crosslinked polyethylene powder such as a non-crosslinked UHMWPE powder.

Similarly, a porous structure of crosslinked polymer (i.e., pre-irradiated) may be placed in a mould, along with polymer powder which is non-crosslinked (i.e., non-irradiated). Thereafter, the two materials are compression moulded to one another under process parameters which cause the non-crosslinked polymer powder to be molten and fused to the porous structure of crosslinked polymer. The composite 30 formed by the fusing of the two materials (i.e., the crosslinked porous structure and the non-crosslinked powder) to one another may be contemporaneously formed into the predetermined shape associated with a prosthetic bearing 10 (e.g., the glenoid bearing 12, the acetabular bearing 14, or the tibial bearing 16). In an exemplary implementation of this process, the crosslinked porous structure is provided as a crosslinked polyethylene porous structure such as a crosslinked UHMWPE porous structure, whereas the non-crosslinked powder is provided as a non-crosslinked polyethylene powder such as a non-crosslinked UHMWPE powder.

Moreover, a porous structure of crosslinked polymer (i.e., pre-irradiated) may be placed in a mould, along with a polymer preform which is non-crosslinked (i.e., non-irradiated). Thereafter, the two materials are compression moulded to one another under process parameters which cause the non-crosslinked polymer preform to be molten and fused to the porous structure of crosslinked polymer. The composite 30 formed by the fusing of the two materials (i.e., the crosslinked porous structure and the non-crosslinked preform) to one another may be contemporaneously formed into the predetermined shape associated with a prosthetic bearing 10 (e.g., the glenoid bearing 12, the acetabular bearing 14, or the tibial bearing 16). In an exemplary implementation of this process, the crosslinked porous structure is provided as a crosslinked polyethylene porous structure such as a crosslinked UHMWPE porous structure, whereas the non-crosslinked preform is provided as a non-crosslinked polyethylene preform such as a non-crosslinked UHMWPE preform.

In regard to fabrication of a composite 30 in which the first polymer layer 32 is constructed of a polymer which has been crosslinked to a first degree and the second polymer layer 34 is constructed of a polymer which has been crosslinked to a second, lesser degree, a number of fabrication processes may also be utilized. Firstly, a preform of polymer crosslinked to a first degree may be placed in a mould, along with a preform of polymer which is crosslinked to a second, lesser degree. Thereafter, the two preforms are compression moulded to one another under process parameters which cause the polymer of the second preform to be molten and fused to the polymer of the first preform. Similarly to as described above, the resultant composite 30 formed by the fusing of the two preforms to one another is contemporaneously formed into the predetermined shape associated with a prosthetic bearing 10 (e.g., the glenoid bearing 12, the acetabular bearing 14, or the tibial bearing 16). In an exemplary implementation of this process, a preform of polyethylene such as UHMWPE which is crosslinked to a first degree is compression moulded to a preform of polyethylene such as UHMWPE which is crosslinked to a second, lesser degree.

Such a composite 30 (i.e., a first polymer layer 32 constructed of a polymer which has been crosslinked to a first degree and a second polymer layer 34 constructed of a polymer which has been crosslinked to a second, lesser degree) may also be fabricated by the use of polymer powders or porous structures. For example, a powder of polymer crosslinked to a first degree may be placed in a mould, along with a preform of polymer which is crosslinked to a second, lesser degree. Thereafter, the two materials (i.e., the powder and the preform) are compression moulded to one another under process parameters which cause the polymer of the preform to be molten and fused with the polymer of the powder. Similarly to as described above, the resultant composite 30 formed by the fusing of the two materials to one another is contemporaneously formed into the predetermined shape associated with a prosthetic bearing 10 (e.g., the glenoid bearing 12, the acetabular bearing 14, or the tibial bearing 16). In an exemplary implementation of this process, a powder of polyethylene such as UHMWPE which is crosslinked to a first degree is compression moulded to a preform of polyethylene such as UHMWPE which is crosslinked to a second, lesser degree.

It should be appreciated that the forms in which the two materials are provided may be swapped. Specifically, a preform of polymer crosslinked to a first degree may be compression moulded with a powder of polymer which is crosslinked to a second, lesser degree in a similar manner to as described above.

Moreover, a powder of polymer crosslinked to a first degree may be placed in a mould, along with a porous structure of polymer which is crosslinked to a second, lesser degree. Thereafter, the two materials (i.e., the powder and the porous structure) are compression moulded to one another under process parameters which cause the polymer of the porous structure to be molten and fused to the polymer of the powder. Similarly to as described above, the resultant composite 30 formed by the fusing of the two materials to one another is contemporaneously formed into the predetermined shape associated with a prosthetic bearing 10 (e.g., the glenoid bearing 12, the acetabular bearing 14, or the tibial bearing 16). In an exemplary implementation of this process, a powder of polyethylene such as UHMWPE which is crosslinked to a first degree is compression moulded with a porous structure of polyethylene such as UHMWPE which is crosslinked to a second, lesser degree.

It should be appreciated that the forms in which the two materials are provided may be swapped. Specifically, a porous structure of polymer crosslinked to a first degree may be compression moulded with a powder of polymer which is crosslinked to a second, lesser degree in a similar manner to as described above.

A similar fabrication process may be utilized in regard to fabrication of a composite 30 having a first polymer layer 32 constructed of a polymer (e.g., polyethylene) and a second polymer layer 34 constructed of an ethylene-acrylate copolymer. In particular, a polymer preform may be placed in a mould, along with a preform of an ethylene-acrylate copolymer. Thereafter, the two preforms are compression moulded to one another under process parameters which cause the copolymer preform to be molten and fused to the polymer preform. Similarly to as described above, the resultant composite 30 formed by the fusing of the two preforms to one another is contemporaneously formed into the predetermined shape associated with a prosthetic bearing 10 (e.g., the glenoid bearing 12, the acetabular bearing 14, or the tibial bearing 16). In an exemplary implementation of this process, the polymer preform is provided as a polyethylene preform such as a UHMWPE preform (crosslinked or non-crosslinked, quenched or non-quenched), whereas the copolymer preform is provided as a PMMA compatible preform.

It should be appreciated that other starting material forms may also be utilized in the fabrication of a composite 30 in which the first polymer layer 32 is constructed of a polymer (e.g., polyethylene) and the second polymer layer 34 is constructed of an ethylene-acrylate copolymer. For example, in addition to preforms, the polymer utilized in the construction of the polymer layer 32 may be provided as either powders or porous structures. Likewise, in addition to preforms, the copolymer utilized in the construction of the polymer layer 34 may be provided as either powders or porous structures.

It should also be appreciated that although the composites 30 have herein been described as having two layers, and have significant advantages thereby in the present invention, other composite configurations are also contemplated. For example, the composite 30 may be configured to include several alternating layers of materials similar to the materials used in regard to the two-layer composites described above. For instance, the composite 30 may be configured to include several (i.e., more than two) layers of alternating crosslinked and non-crosslinked UHMWPE.

Moreover, it may be desirable to use vacuum moulding for some materials. For example, vacuum moulding may be preferred where one or more of the layers comprises a non-quenched material.

Advantageously, the tensile properties associated with the interfaces between the layers 32, 34 are as strong as at least the weaker of the individual layers 32,34. Such interfacial tensile properties may be evaluated in an interfacial tensile test as a gauge for composite structure integrity. Type V tensile specimens of 400 micron thickness were prepared from various composites and were tested based on the ASTM D638 standard. The interface section was placed within the gauge length of the narrow section of the tensile test specimen.

**Table I : Interfacial tensile properties of two crosslinked UHMWPE composite mouldings**

| Composite structure | Composition | Ultimate tensile strength | Elongation |
|---|---|---|---|
| Preform I | Non-irradiated GUR 1020 preform | 55.98 ± 6.55 MPa | 397 ± 55% |
| Preform II | 50 kGy annealed GUR 1050 preform | (8.12 ± 0.95 ksi) | |
| Powder | GUR 1020 powder | 47.5 ± 1.72 MPa | 403 ±16% |
| Preform | 50 kGy annealed GUR 1050 preform | (6.89 ± 0.25 ksi) | |

**Table II Tensile properties of pre-consolidated UHMWPE material**

| Material | Ultimate tensile strength | Elongation |
|---|---|---|
| 50 kGy annealed GUR 1050 preform | 52.12 ± 3.38 MPa | 301 ±23% |
| (ram extruded) | (7.56 ± 0.49 ksi) | |
| Non-irradiated GUR 1020 | 61.98 ± 4.55 MPa | 475 ±26% |
| (ram extruded) | (8.99 ± 0.66 ksi) | |

As shown in Tables I and II, interfacial ultimate tensile strength data indicate that the interfaces of composites 30 have an integrity which is approximately comparable to the fully consolidated or fully fused forms of the starting materials (i.e. the materials of which the individual layers 32, 34 are constructed). Moreover, the corresponding data for elongation at break at the interfaces for the composites 30 are at least as good as the weaker of the two starting materials.

In the composite structures of Table I, the two polymer layers 32, 34 have been fused by heating the two layers to cause the adjacent portions of both polymer layers to become molten while compression moulding the layers into the composite. It should also be understood that other processes can be used to create such a melt-fused interface between the polymer layers. For example, welding can be used to create a melt-fused interface. It is believed that with melt-fusion, the polymer chains from each polymer layer become intermingled and entangled to create the strong interface, with strong bonding between the layers. Generally, the expressions "melt-fusion" and "melt-fused" are used herein to denote interfaces between polymer layers wherein the parts of both polymer layers at the interface have been melted.

It is expected that other methods of securing the two polymer layers can be used for some applications. For example, instead of melt-fusion, it is expected that mechanical interlocks can be used in some applications. With the choice of appropriate materials and processes, mechanical interlocks between polymer layers may provide an interface with adequate mechanical and dynamic properties for the composite to be used as an implantable bearing for an orthopaedic prosthesis.

For an application relying upon mechanical interlocks, it is anticipated that mechanical interlocking with adequate interfacial strength can be achieved by providing a second layer of polymer 34 comprising a porous structure of a high-temperature engineering polymer, such as one from the polyaryletherketone family or the polyimide family, and by control of process parameters. In such an application, a crosslinked UHMWPE layer may be used for the first layer of polymer 32 for the articulating surface. The crosslinked UHMWPE layer 32, in the form of a powder or preform, may be compression moulded to the layer 34 of porous high temperature engineering polymer under a temperature that will melt at least a portion of the UHMWPE layer, so that UHMWPE melts into and fills some of the pores of the high temperature engineering material; when this UHMWPE material solidifies, the two polymer layers will be mechanically bonded together.

The compression moulding can be done at a temperature high enough to melt the UHMWPE layer but below the melting point of the second layer of polymer 34. The high temperature may be localized at the interface of the layers 32, 34. The porous structure may comprise a solid section for machining into the final geometry. The structural integrity of the interface in this composite embodiment relies on mechanical interlocking of the two polymer layers. It is also anticipated that various mechanical locking mechanisms can be used, provided that materials and process parameters are selected to provide the desired strength at the interface of the polymer layers 32, 34.

The layer of porous high temperature engineering polymer may comprise a engineering polymer such as polyetheretherketone, polyetherketone, polyetherketoneetherketoneketone or polyimide. These materials are biocompatible and are able to withstand the processing temperature for UHMWPE without significant deformation. Required preform or porous structures can be readily fabricated from these raw materials using conventional processing techniques. Although it is expected that these polymer materials will be useful as one of the polymer layers when relying upon a mechanical interlock, the present invention is not limited to these materials unless the claims expressly call for them. The present invention may also encompass newly developed polymers, unless a particular polymer is expressly set forth in the claims.

In addition, although the mechanical interlock that secures the two polymer layers together can be formed by compression moulding the two polymer layers together, methods such as hot isostatic pressing may be used to secure the two layers of polymer 32, 34 together with a mechanical interlock. In addition, as new polymer materials are developed, new methods of securing the polymer layers together may also be developed. Accordingly, the present invention is not limited to any particular means of securing the polymer layers together, and may encompass newly developed materials and securing means, unless a particular material or process is expressly set forth in the claims.

In addition to ultimate tensile strength and elongation, the interface of the two secured polymer layers in the composite bearing is expected to also have other mechanical and dynamic properties comparable to the other mechanical properties of at least the weaker of the fully fused or fully consolidated forms of the polymer material in the two layers. Mechanical and dynamic properties of the interface include not only ultimate tensile strength, yield strength, elongation to break, and modulus, but also oxidation resistance, impact strength, compression strength, shear strength and fatigue strength (as shown through different forms of fatigue testing, e.g., three-point bending, tension, compression and flex fatigue). "Comparable" is meant to include no statistical difference in properties, as well as overlaps in data points, as in the case of the ultimate tensile strength of the Powder-Preform Composite Structure of Table I and the 50 KGy, Annealed GUR 1050 Ram Extruded material in Table II, for example.

It should be understood that unless specific mechanical and dynamic properties are expressly called for in the claims, the invention is not intended to be limited to composites with interfaces with any particular mechanical or dynamic property. Although the composite article should have properties suitable for an orthopaedic implant, the properties at the interface may not need to be comparable to the mechanical and dynamic properties of the fully consolidated materials of the layers. For example, if the two polymer layers are made of materials with great enough shear strength, then the shear strength of the interface may be less significant.

The composite articles with layers secured through a melt-fusion process possess may be preferred over those with layers secured through mechanical interlocks. The temperature of the melt-fusion process should be sufficient to destroy any contaminants at the pre-fusion interface. In addition, melt-fusion results in an interface where the two polymers are in intimate contact, with no pore, void, gap, crack or separation that would allow for the ingress of agents that would compromise sterilization. Therefore, surface sterilization should be adequate for the melt-fused polymer composite implants. The ability to use surface sterilization techniques, such as gas plasma sterilization and ethylene oxide sterilization, instead of irradiation, is advantageous when an UHMWPE material is used as one of the polymer layers. Gamma irradiation sterilization of UHMWPE material can generate free-radicals and can make the material more susceptible to oxidation.

For polymer composite bearings with polymer layers secured through mechanical interlocks, some gap or separation may continue to exist between the layers after they are secured together, since only one of the polymer layers will be melted. If the gap or separation is large enough, agents that could compromise sterilization could enter the gap. Accordingly, unless special processing techniques are employed, sterilization could require gamma irradiation to reach the level of the interface of the polymer layers. In addition, if either polymer layer exists as a porous structure after the polymer layers are secured together, proper sterilization will probably require gamma irradiation.

If it is desired to avoid gamma irradiation sterilization of composites with mechanical interlocks, known special processing techniques may be employed to ensure a sterile environment, such as processing in a clean room.

In addition, new sterilization techniques may be developed, and new materials may be developed, or materials may be selected that do not degrade under gamma irradiation sterilization. In either event, mechanical interlocking polymer layers may be preferred.

In addition to the ability to surface sterilize the melt-fused composite bearings of the present invention, the prosthetic bearings 10 and associated methods for making the same have a number of advantages over heretofore designed bearings and associated methods. For example, by constructing the bearing as a composite (i.e., the composite 30), the materials selected with each layer of the composite may be selected to provide the desired mechanical and dynamic properties. In particular, as described above, the layer in which the articulating surface 18 of the bearing 10 is defined may be constructed with materials which have favourable mechanical and dynamic properties for use as an articulating surface (e.g., high wear and oxidation resistance). On the other hand, the layer in which the engaging surface 20 is defined may be constructed with materials which have favourable mechanical and dynamic properties for use as an engaging surface (e.g., high ductility, toughness and creep resistance).

Moreover, use of an ethylene-acrylate copolymer as an underlying layer provides the bearings 10 of the present invention with advantages over heretofore designed composite bearings. Firstly, such a copolymer has a component for both (1) providing suitable adhesion to the polyethylene-based upper layer, and (2) providing suitable adhesion to bone cement. Other attempts at providing this functionality have been met with limited success since, amongst other things, they have attempted to utilize dry blends of materials (as opposed to an actual copolymer), and, as a result, have been subjected to both process and product variations.

Although the crosslinked polymer or more highly crosslinked polymer may typically be used for the articulating surface of the composite with non-crosslinked or less crosslinked polymer being used for the engaging surface, there may be instances where it is desirable for the crosslinked polymer or more highly crosslinked polymer layer to be used for the engaging surface and the non-crosslinked or less crosslinked polymer to be used for the articulating surface.

## Claims

1. A method of making a bearing for an orthopaedic prosthesis, which comprises:
a. exposing a first polymer to a dose of radiation so as to cause the first polymer to become crosslinked,
b. securing a layer (32) of the first polymer, after it has been crosslinked, to a layer (34) of a second polymer to form a composite structure which comprises the layers of the first and second polymers, in which the first layer is secured to the second layer by a process which comprises compression moulding at elevated temperature and pressure sufficient to cause the polymer layers to become bonded to one another, and
c. forming the composite into a predetermined shape.

2. A method as claimed in claim 1, which includes a step of exposing the second polymer to a dose of radiation so as to cause the second polymer to become crosslinked, in which the dose of radiation to which the second polymer is exposed is different from the dose of radiation to which the first polymer is exposed.

3. A method as claimed in claim 2, in which the dose of radiation to which the second polymer is exposed is less than the dose of radiation to which the first polymer is exposed.

4. A method as claimed in claim 2, in which the degree of crosslinking of the first polymer is greater than the degree of crosslinking of the second polymer.

5. A method as claimed in claim 1, in which the securing and forming steps are performed simultaneously.

6. A method as claimed in claim 1, in which the first polymer is in powder form during the step of exposing it to radiation.

7. A method as claimed in claim 1, in which the first polymer is in the form of a preform during the step of exposing it to radiation and when secured to the layer of the second polymer.

8. A method as claimed in claim 1, in which the second polymer is in the form of a preform when secured to the layer of the first polymer.

9. A method as claimed in claim 8, in which the layer (34) of the second polymer is porous.

10. A method as claimed in claim 1, in which the securing step involves subjecting the first and second polymers to elevated temperature and pressure which are sufficient to form a melt fused interface in which both polymer layers (32, 34) at the interface have been melted.

11. A method as claimed in claim 1, in which the layer (34) of the second polymer is porous, and in which the securing step involves subjecting the first polymer to elevated temperature and pressure which are sufficient to cause it to penetrate the porous structure of the second polymer so that the layers (32, 34) of the first and second polymers become mechanically bonded together.

## Patentansprüche

1. Verfahren zur Herstellung eines Lagers für eine orthopädische Prothese, das umfaßt:
a. Aussetzen eines ersten Polymers einer Strahlendosis, damit das Polymer vernetzt wird,
b. Befestigen einer Schicht (32) aus dem ersten Polymer, nachdem es vernetzt worden ist, an einer zweiten Schicht (34) aus einem zweiten Polymer zur Bildung einer Komposit-Struktur, die die Schicht aus dem ersten Polymer und die Schicht aus dem zweiten Polymer umfaßt, wobei die erste Schicht an der zweiten Schicht durch einen Prozeß befestigt wird, der Formpressen auf erhöhter Temperatur und bei einem Druck umfaßt, der ausreicht, damit die Polymerschichten aneinandergeklebt werden, und
c. Formen des Komposits in einer vorab festgelegten Form.

2. Verfahren nach Anspruch 1, das einen Schritt des Aussetzens des zweiten Polymers einer Strahlendosis enthält, damit das zweite Polymer vernetzt wird, wobei die Strahlendosis, der das zweite Polymer ausgesetzt wird, anders als die Strahlendosis ist, der das erste Polymer ausgesetzt wird.

3. Verfahren nach Anspruch 2, in dem die Strahlendosis, der das zweite Polymer ausgesetzt wird, geringer als die Strahlendosis ist, der das erste Polymer ausgesetzt wird.

4. Verfahren nach Anspruch 2, in dem der Grad der Vernetzung des ersten Polymers größer als der Grad der Vernetzung des zweiten Polymers ist.

5. Verfahren nach Anspruch 1, in dem der Befestigungsschritt und der Formschritt simultan durchgeführt werden.

6. Verfahren nach Anspruch 1, in dem das erste Polymer in Pulverform während des Schrittes des Aussetzens einer Strahlung vorliegt.

7. Verfahren nach Anspruch 1, in dem das erste Polymer in Form einer Vorform während des Schrittes des Aussetzens einer Strahlung und bei Befestigung an der Schicht aus dem zweiten Polymer vorliegt.

8. Verfahren nach Anspruch 1, in dem das zweite Polymer in Form einer Vorform bei Befestigung an der Schicht aus dem ersten Polymer vorliegt.

9. Verfahren nach Anspruch 8, in dem die Schicht (34) aus dem zweiten Polymer porös ist.

10. Verfahren nach Anspruch 1, in dem der Befestigungsschritt mit dem Aussetzen des ersten Polymers und des zweiten Polymers einer erhöhten Temperatur und einem Druck verbunden ist, der ausreicht, um eine schmelzverschweißte Grenzfläche zu bilden, wobei beide Polymerschichten (32, 34) an der Grenzfläche geschmolzen worden sind.

11. Verfahren nach Anspruch 1, in dem die Schicht (34) aus dem zweiten Polymer porös ist und in dem der Befestigungsschritt mit dem Aussetzen des ersten Polymers einer erhöhten Temperatur und einem Druck verbunden ist, der ausreicht, um in die poröse Struktur des zweiten Polymers einzudringen, so daß die Schicht (32, 34) aus dem ersten Polymer und dem zweiten Polymer miteinander mechanisch verbunden werden.

## Revendications

1. Procédé de fabrication d'un palier pour une prothèse orthopédique, comprenant les étapes consistant à:
a) exposer un premier polymère à une dose de rayonnement de façon à provoquer la réticulation du premier polymère,
b) ancrer une couche (32) du premier polymère, après sa réticulation, dans une couche (34) d'un deuxième polymère pour former une structure composite qui comprend les couches des premier et deuxième polymères, dans lequel la première couche est ancrée dans la deuxième couche par un procédé qui comprend un moulage par compression à température et pression élevées, suffisantes pour provoquer la liaison des couches de polymère, et
c) former le composite selon une forme prédéterminée.

2. Procédé selon la revendication 1, incluant une étape consistant à exposer le deuxième polymère à une dose de rayonnement de façon à provoquer la réticulation du deuxième polymère, dans lequel la dose de rayonnement à laquelle est exposé le deuxième polymère est différente de la dose de rayonnement à laquelle est exposée le premier polymère.

3. Procédé selon la revendication 2, dans lequel la dose de rayonnement à laquelle est exposé le deuxième polymère est inférieure à la dose de rayonnement à laquelle est exposé le premier polymère.

4. Procédé selon la revendication 2, dans lequel le degré de réticulation du premier polymère est supérieur au degré de réticulation du deuxième polymère.

5. Procédé selon la revendication 1, dans lequel les étapes d'ancrage et de formation sont effectuées simultanément.

6. Procédé selon la revendication 1, dans lequel le premier polymère se présente sous forme de poudre pendant l'étape de son exposition à un rayonnement.

7. Procédé selon la revendication 1, dans lequel le premier polymère se présente sous la forme d'une préforme pendant l'étape de son exposition à un rayonnement et lorsqu'il est ancré dans la couche du deuxième polymère.

8. Procédé selon la revendication 1, dans lequel le deuxième polymère se présente sous la forme d'une préforme lorsqu'il est ancré dans la couche du premier polymère.

9. Procédé selon la revendication 8, dans lequel la couche (34) du deuxième polymère est poreuse.

10. Procédé selon la revendication 1, dans lequel l'étape d'ancrage implique le fait de soumettre les premier et deuxième polymères à une température et une pression élevées qui sont suffisantes pour former une interface fondue dans laquelle les deux couches de polymère (32, 34) au niveau de l'interface ont été fondues.

11. Procédé selon la revendication 1, dans lequel la couche (34) du deuxième polymère est poreuse, et dans lequel l'étape d'ancrage implique le fait de soumettre le premier polymère à une température et une pression élevées qui sont suffisantes pour l'amener à pénétrer dans la structure poreuse du deuxième polymère de sorte que les couches (32, 34) des premier et deuxième polymères sont amenées à se lier mécaniquement ensemble.
